## Europäisches Patentamt

## European Patent Office

## Office européen des brevets

(19)

(11) Veröffentlichungsnummer: **0 030 710**
**B1**

(12)

# EUROPÄISCHE PATENTSCHRIFT

(45) Veröffentlichungstag der Patentschrift:
12.03.86

(51) Int. Cl.⁴: **G 01 N 33/80**, G 01 N 33/96

(21) Anmeldenummer: **80107786.8**

(22) Anmeldetag: **11.12.80**

(54) Verfahren zur Stabilisierung von Lymphozyten/Thrombozytenmischsuspensionen.

(30) Priorität: **12.12.79 DE 2949824**

(43) Veröffentlichungstag der Anmeldung:
**24.06.81 Patentblatt 81/25**

(45) Bekanntmachung des Hinweises auf die Patenterteilung:
**12.03.86 Patentblatt 86/11**

(84) Benannte Vertragsstaaten:
**AT BE CH DE FR GB IT LI LU NL SE**

(56) Entgegenhaltungen:
**DE - A - 2 546 166**
**DE - A - 2 727 730**
**US - A - 3 714 345**

**BRITISH JOURNAL OF HAEMATOLOGY, Band 39, 1978, VON DEM BORNE et al. "A simple immunofluorescence test for the detection of platelet antibodies", Seiten 195-207**
**ÄRZTLICHES LABORATORIUM, Band 20, 1974, M. SEHRBUNDT et al. "Fluoreszenzoptischer Nachweis antilymphozytärer und antithrombozytärer Antikörper", Seiten 354-362**
**LAB MED 7 (1983), 312-317**

**Die Akte enthält technische Angaben, die nach dem Eingang der Anmeldung eingereicht wurden und die nicht in dieser Patentschrift enthalten sind.**

(73) Patentinhaber: **Sehrbundt, Marianne, Dr.,**
**Max-Bruch-Strasse 6, D-5000 Köln-Lindenthal (DE)**

(72) Erfinder: **Sehrbundt, Marianne, Dr.,**
**Max-Bruch-Strasse 6, D-5000 Köln-Lindenthal (DE)**

(74) Vertreter: **Werner, Hans-Karsten, Dr. et al,**
**Deichmannhaus am Hauptbahnhof, D-5000 Köln 1 (DE)**

## Beschreibung

Gegenstand der vorliegenden Erfindung ist ein Verfahren zur Stabilisierung von Lymphozyten/ Thrombozytenmischsuspensionen, die zur routinemässigen fluorometrischen Bestimmung von Lymphozyten- und Thrombozytenantikörpern in Patienten-Seren geeignet sind.

Nach wie vor steht für den transfundierenden Arzt die hämolytische Transfusionsreaktion wegen ihrer Gefährlichkeit im Vordergrund, obwohl sie von der Zahl her nicht mehr an erster Stelle rangiert. Seit Jahren richtet sich das Augenmerk mehr auf die häufigeren Transfusionsreaktionen, die durch Leukozyten und Thrombozyten hervorgerufen werden. Sie führen klinisch zu Sofortreaktionen oder febrilen Spätreaktionen, die mit einer Transfusionspurpura kombiniert sein können. Stehen keine kompatiblen Thrombozyten zur Verfügung, können lebensbedrohliche Blutungen eintreten. Es sind bereits eine Reihe von Verfahren zum Nachweis antileukozytärer und antithrombozytärer Antikörper beschrieben worden. In der Praxis eingeführt haben sich praktisch nur fluoreszenzoptische Tests; vgl. Ärztliches Laboratorium 20, S. 354–362 (1974) und British Journal of Haematology, 1978, 39, Seite 195 bis 207.

Eine Verträglichkeitsprobe für Leukozyten und Thrombozyten vor der Transfusion hat dadurch an Bedeutung zugenommen, dass bei zunehmender Zytostase-Therapie mit der damit verbundenen iatrogenen Knochenmarksdepression immer häufiger Patienten auf eine unterstützende Therapie mit Thrombozyten und Leukozyten angewiesen sind. Entsprechende Tests sind auch für Nieren und Knochenmarkstransplantationen erforderlich.

Die bisher vorliegenden Routinetests haben nur eine begrenzte Aussagefähigkeit. Die Mischsuspensionen von Lymphozyten und Thrombozyten für die fluorometrische Messung mussten bisher stets neu gewonnen werden und konnten daher schon aus technischen Gründen nicht in dem Masse durchgeführt werden, wie medizinisch zu fordern wäre. Um eine ausreichende Informationsbreite zu erhalten, ist bei den bisher üblichen Tests die Untersuchung des Patientenserums mit 4 verschiedenen Testverfahren (Leukozyten-, Thrombozyten-, Agglutinationstest, Lymphozytotoxizitätstest und Thrombozyten-Mikro-Komplementbindungs-Reaktion) mit Hilfe von 3 verschiedenen, jeweils frisch zu gewinnenden Zellsuspensionen und einer nur über 3 Monate stabilisierbaren Thrombozyten-Suspension erforderlich.

Der Vorteil der Immunfluoreszenz besteht darin, dass eine Antigen-Antikörperbindung unmittelbar an der Zellmembran sichtbar gemacht werden kann, unabhängig davon, ob es sich um einen kompletten oder inkompletten, komplementbindenden oder nichtkomplementbindenden Antikörper handelt. Die Immunfluoreszenz ist darüber hinaus wesentlich empfindlicher als die bisher üblichen Verfahren, jedoch neigt sie zu unspezifischen, falsch positiven Fluoreszenzerscheinungen. Die Vor- und Nachteile der fluorometrischen Methoden und ihre bisherigen Anwendungsgebiete sind im Brit. Journal Haematol. 36, Seite 533 bis 544, 1977 zusammengefasst. Aus dieser und den weiter oben zitierten Arbeiten ist bekannt, dass man bei frisch gewonnenen Thrombozyten mit Formaldehyd unspezifische Fluoreszenz unterdrücken kann, was auf Zytoadherenz von Plasmaproteinen zurückgeführt wird. Diese falsch positiven Ergebnisse werden durch einprozentige Formaldehydlösungen unterdrückt. Die Autoren sind der Meinung, dass eine Lagerung der Formaldehyd fixierten Thrombozyten über Nacht bei 4 °C die Nachweisbarkeit von Thrombozytenantigenen bereits beeinträchtigt (Seite 197). Auf Seite 198 ist eine Zweitfixierung mit Formaldehyd beschrieben, die erst nach der Antikörperbindung stattfindet. Diese Zweitfixierung entspricht der im Test gemäss Ärztelabor 20 beschriebenen Fixierung mit 95%igen Alkohol. In der Praxis hat sich für diese Fixierung auch Aceton bewährt.

In der DE-A-2 727 730 ist eine konservierte, lösliche, nicht koagulierende Blutzusammensetzung beschrieben, die für die fluorometrische Methode völlig ungeeignet ist. Die Stabilisierung nach dem dort beschriebenen Verfahren dient nur noch der Zählung der verschiedenen Blutkörperchen in einem automatischen Zählgerät und bedeutet eine Stabilisierung der Blutproben für 24 bis 72 Stunden. Die nach dem dort beschriebenen Verfahren erhaltenen Lymphozyten sind dem Formaldehyd so lange ausgesetzt, dass sie als «konservierte Leichen» vorliegen. Nach dem erfindungsgemässen Verfahren hingegen soll nur die Oberfläche der Lymphozyten und auch der Thrombozyten so weit verändert werden, dass ohne Zerstörung der Zellwand und der Lebensfähigkeit die Lebensdauer verlängert wird, jedoch die spezifische Bindungsreaktion unterdrückt wird und dennoch die spezifische fluorometrische Bestimmung der spezifisch erkennbaren Oberflächenstrukturen voll erhalten bleibt.

Auch die DE-A-2 546 166 betrifft nur ein Verfahren zur Stabilisierung von Thrombozyten über einen längeren Zeitraum mit den Gerbstoffen. Selbst wenn aus «British J. of Haematology», 1978, Band 39, Seite 195, sowie aus der DE-A-2 546 166 bekannt war, dass eine Behandlung von Formaldehyd die auf den Blutkörperchenoberflächen befindlichen Antigene in ihren immunologischen Eigenschaften nicht verändert, so dass sie auch noch zur fluorometrischen Bestimmung geeignet sind, war doch nicht vorherzusehen, dass bereits eine kurzfristige Behandlung mit Formaldehyd ohne Zerstörung der Zellwand und der Lebensfähigkeit die Lebensdauer der Lymphozyten und Thrombozyten erheblich verlängern würde.

Gegenstand der vorliegenden Erfindung ist somit ein Verfahren zur Stabilisierung von Lymphozyten/Thrombozytenmischsuspensionen, die zur routinemässigen fluorometrischen Bestimmung von Lymphozyten- und Thrombozytenantikörpern in Patientenseren geeignet sind, dadurch gekennzeichnet, dass man die frisch hergestellten Mischsuspensionen mit 1 bis 5%igen Formaldehydlösungen bei 4 bis 30 °C in Gegenwart einer neutralen phosphatgepufferten Kochsalzlösung umsetzt, den Formaldehydpuffer auswäscht und die Suspension in einem Lösungsmittel lagert.

Zur Durchführung des erfindungsgemässen Verfahrens setzt man die frischgewonnene Lymphozyten/Thrombozytenmischsuspension mit einer 1 bis 5%igen, vorzugsweise 3 bis 4%igen Formaldehydlösung um. Die Reaktionstemperatur wird von 4 bis 30 °C gewählt. Vorzugsweise arbeitet man bei Raumtemperatur. Der Formaldehyd wird mit einer neutralen phosphatgepufferten Kochsalzlösung verdünnt, welche nach der Umsetzung wieder ausgewaschen werden kann. Die Umsetzungsdauer der Mischsuspension mit der Formaldehydlösung hängt von der Konzentration und Reaktionstemperatur ab. Bei den bevorzugten Reaktionsbedingungen genügt eine Umsetzungszeit von 3 bis 10, vorzugsweise 5 Minuten.

Zur Lagerung überführt man die Suspension in ein geeignetes Lösungsmittel. Hanks-Lösung hat sich besonders bewährt. Bei 4 °C ist eine derartig stabilisierte Suspension in Hanks-Lösung viele Wochen lagerfähig, ohne dass merklicher Zellabbau zu beobachten ist.

Da Hanks-Lösung ein guter Bakterien-Nährboden ist, empfiehlt es sich für die Praxis, zusätzlich ein Bakteriostatikum zuzusetzen, wobei übliche Zusätze wie Natriumacid oder Penicillin infrage kommen.

Für die praktische Handhabung im routinemässigen Verfahren füllt man die stabilisierte Lymphozyten/Thrombozytenmischsuspension beispielsweise in der Hanks-Lösung in Panel-Fläschchen und stellt sie zu Serien von Suspensionen mit verschiedenen Antigenmustern zusammen. In dieser Form können sie leicht versandt und im Routinelabor in einfacher Weise eingesetzt werden. Frische Mischsuspensionen von Lymphozyten und Thrombozyten kann man beispielsweise nach der im Ärztlichen Laboratorium loc. cit. beschriebenen Methode mit Lymphoflot herstellen. Vorzugsweise wird man jedoch die inzwischen bewährten Zellseparatoren (z.B. Hemonetics Modell 30) benutzen. Mit diesen Zellseparatoren ist es inzwischen möglich, aus dem Blut eines Spenders die Leukozyten (überwiegend Lymphozyten) und Thrombozyten durch Zentrifugation im geschlossenen System zu separieren und abzuschöpfen. Plasma und Erythrozyten werden dem Spender sofort retransfundiert. Auf diese Weise kann man z.B. pro Spender $5 \times 10^{11}$ Thrombozyten und ca. $3 \times 10^9$ Lymphozyten entnehmen. Diese Menge reicht für etwa 10 000 Tests aus. Da in einem Panel-Fläschchen normalerweise die Menge für 100 Testansätze enthalten ist, kann mit einer Spende der Bedarf von etwa 100 Panel-Fläschchen erhalten werden.

Die durch den Zellseparator gewonnene «Buffy-Coat»-Schicht des Blutes wird mit Hilfe der Dichtegradienten-Zentrifugation von Erythrozyten, Macrophagen u.ä. befreit. Die Lymphozyten und Thrombozyten werden dann auf eine für den Fluoreszenztest optimale Zellkonzentration und das geeignete Verhältnis von Thrombozyten zu Lymphozyten eingestellt. Diese so standardisierten Lösungen können dann stabilisiert werden. Prinzipiell ist es auch möglich, erst zu stabilisieren und die Standardkonzentrationen einzustellen. Für einen kompletten Satz Panel-Fläschchen wird man 12 bis 20 Spender auswählen, die somit das gewünschte Antigenmuster liefern.

Es ist ohne weiteres einzusehen, dass derartig serienmässig hergestellte und standardisierte Sätze von Panel-Fläschchen wesentlich einfacher und kostengünstiger herstellbar sind, als wenn für jede Testserie die Mischsuspensionen frisch hergestellt werden müssen. Die Herstellung einer frischen Mischsuspension erfordert mindestens 3 Stunden Arbeitszeit einer hochqualifizierten Fachkraft, während im industriellen Massstab sehr rasch auch angelernte Fachkräfte eingesetzt werden können. Nicht stabilisierte Thrombozyten sind erfahrungsgemäss maximal 8 Tage und frisch zubereitete Leukozyten nur einige Stunden bis maximal 3 bis 4 Tage stabil und müssen dann verworfen werden. Erst die erfindungsgemässe Stabilisierung ermöglicht es, Lymphozyten/Thrombozytenmischsuspensionen im grösseren Massstab an zentralen Stellen herzustellen und an die jeweiligen Laboratorien zu versenden. Hierdurch wird die an sich medizinisch schon lange geforderte ausreichende Vorerprobung auf Verträglichkeit einer Transfusionslösung auch die leukozytär/thrombozytären Systeme betreffend auf breiter Basis ermöglicht.

In den nachfolgenden Beispielen ist das erfindungsgemässe Verfahren näher erläutert.

Beispiel 1
Gewinnung von frischen Mischsuspensionen von Lymphozyten und Thrombozyten

Unter Verwendung des Hemonetics Modell 30 Zellseparators werden von HLA-typisierten Spendern im Durchschnitt ca $5 \times 10^{11}$ Thrombozyten und ca. $3 \times 10^9$ Lymphzyten gewonnen. Es wird die so gewonnene «Buffy-Coat»-Schicht des Blutes mit Hilfe einer Dichtegradienten-Zentrifugation von Erythrozyten, Macrophagen u.ä. befreit. Die so erhaltenen Mischsuspensionen sind im allgemeinen nur einige Stunden, maximal jedoch wenige Tage, im gekühlten Zustand haltbar. Eine Konservierung durch Tieffrieren ist aufwendig und führt bereits zu Vorschädigungen der Zellen beim Frieren und Wiederauftauen. Für zuverlässige Vergleichsmessungen sind sie maximal 48 bis 72 Stunden verwendbar.

Stabilisierung durch Formaldehyd
Ein Teil 37-%ige Formaldehydlösung (Merck, Art. Nr. 4002) und 10 Teile phosphatgepufferte Kochsalzlösung (pH 7,2) werden miteinander vermischt. In dieses Gemisch wird eine frischbereitete Lymphozyten/Thrombozytenmischsuspension bei Raumtemperatur eingegeben und 5 Minuten stehengelassen. Nach dem Abzentrifugieren wird mit Hanks-Lösung (Seromed, Art. Nr. 2015) einmal ausgewaschen und dann hierin suspendiert. Diese Suspension ist bei 4 °C über viele Wochen ohne grossen Zellverlust haltbar und kann sofort im Fluoreszenztest eingesetzt werden.

## Beispiel 2

Zu vergleichbaren Ergebnissen kommt man bei Verwendung von Formaldehydlösungen mit phosphatgepuffertem Kochsalz im Verhältnis 1:8 bis 1:20. Die Reaktionszeit kann durch höhere Formaldehydkonzentrationen und höhere Temperaturen verkürzt und durch niedrigere Konzentrationen an Formaldehyd und niedrigere Temperaturen verlängert werden. Die optimalen Bedingungen können an die Bedürfnisse der Praxis bei grösseren Ansätzen entsprechend angepasst werden.

## Beispiel 3

Standardisierung und Abfüllung in Panel-Fläschchen

Bei grösseren Abweichungen des Verhältnisses Thrombozyten zu Lymphozyten können durch längeres Zentrifugieren lymphozyten- bzw. thrombozytenreichere Fraktionen hergestellt werden, mit denen die jeweils gewonnene Suspension verschnitten und damit standardisiert werden kann. Die Suspension in der Hanks-Lösung wird auf die jeweils gewünschte Standardmenge Thrombozyten und Lymphozyten eingestellt und dann in Sätze von Panel-Fläschchen abgefüllt. Nach dem Etikettieren werden die Fläschchen zu Sets von Panel-Fläschchen zusammengestellt und bei 4 °C gelagert. Sie können in dieser Form auch versandt und an die Endverbraucher verteilt werden.

## Patentansprüche

1. Verfahren zur Stabilisierung von Lymphozyten/Thrombozytenmischsuspensionen, die zur routinemässigen fluorometrischen Bestimmung von Lymphozyten- und Thrombozytenantikörpern in Patienten-Seren geeignet sind, dadurch gekennzeichnet, dass man die frisch hergestellten Mischsuspensionen mit 1 bis 5%igen Formaldehydlösungen bei 4 bis 30 °C in Gegenwart einer neutralen phosphatgepufferten Kochsalzlösung umsetzt, den Formaldehydpuffer auswäscht und die Suspension in einem Lösungsmittel lagert.

2. Verfahren zur Stabilisierung von Lymphozyten/Thrombozytenmischsuspensionen nach Anspruch 1, dadurch gekennzeichnet, dass man die Suspension in Hanks-Lösung bei 4 °C lagert.

3. Verfahren zur Stabilisierung von Lymphozyten/Thrombozytenmischsuspensionen nach Anspruch 1 oder 2, dadurch gekennzeichnet, dass man der stabilisierten Suspension ein Bakteriostatikum zusetzt.

4. Verfahren zur Stabilisierung von Lymphozyten/Thrombozytenmischsuspensionen nach einem der Ansprüche 1 bis 3, dadurch gekennzeichnet, dass man die Suspensionen in standardisierter Form in Panelfläschchen abfüllt und zu Serien verschiedener Antigenmuster zusammenstellt.

## Claims

1. A method for stabilizing lymphocyte/thrombocyte mixed suspensions suitable for the fluorometric routine determinations of lymphocyte and thrombocyte antibodies in the serum of a patient, characterized in that the freshly prepared mixed suspensions are reacted with 1 to 5% formaldehyde solutions at from 4 °C to 30 °C in the presence of a neutral phosphatebuffered saline, the formaldehyde-buffer is washed out and the suspension is stored in a solvent.

2. The method for stabilizing lymphocyte/thrombocyte mixed suspensions according to claim 1, characterized in that the suspension is stored in Hanks' solution at 4 °C.

3. The method for stabilizing lymphocyte/thrombocyte mixed suspensions according to claims 1 or 2, characterized in that a bacteriostatic agent is added to the stabilized suspension.

4. The method for stabilizing lymphocyte/thrombocyte mixed suspensions according to anyone of claims 1 to 3, characterized in that the suspensions in a standardized form are filled into small Panel bottles and combined to give sets of various antigen patterns.

## Revendications

1. Procédé de stabilisation de suspensions mixtes lymphocytes thrombocytes destinées à la détermination fluorimétrique courante d'anticorps antilymphocytaires et antithrombocytaires dans le sérum de patients, caractérisé par le fait que l'on fait réagir les suspensions mixtes fraîchement préparées sur des solutions de formaldéhyde à 1 à 5%, à 4 à 30 °C, en présence d'une solution neutre de chlorure de sodium tamponnée ou phosphate, que l'on élimine par lavage le tampon ou formaldéhyde et que l'on conserve la suspension dans un solvant.

2. Procédé de stabilisation de suspensions mixtes lymphocytes/thrombocytes selon la revendication 1, caractérisé par le fait que l'on conserve la suspension dans de la solution de Hanks à 4 °C.

3. Procédé de stabilisation de suspensions mixtes lymphocytes/thrombocytes selon l'une des revendications 1 et 2, caractérisé par le fait que l'on ajoute un bactériostatique à la suspension stabilisée.

4. Procédé de stabilisation de suspensions mixtes lymphocytes/thrombocytes selon l'une des revendications 1 à 3, caractérisé par le fait que l'on introduit les suspensions sous forme normalisée dans des flacons de Panel et qu'on les rassemble en série ayant différents groupes antigéniques.